(19) Europäisches Patentamt
European Patent Office
Office européen des brevets

(11) Veröffentlichungsnummer: **0 087 385**
**B1**

# (12) EUROPÄISCHE PATENTSCHRIFT

(45) Veröffentlichungstag der Patentschrift: **17.09.86**

(51) Int. Cl.[4]: **C 08 G 69/26**, C 08 F 283/04, C 08 F 2/50, C 08 J 3/24, C 08 K 5/00

(21) Anmeldenummer: **83810030.3**

(22) Anmeldetag: **24.01.83**

## (54) Vernetzbare transparente Polyamide.

(30) Priorität: **29.01.82 CH 561/82**

(43) Veröffentlichungstag der Anmeldung:
**31.08.83 Patentblatt 83/35**

(45) Bekanntmachung des Hinweises auf die Patenterteilung:
**17.09.86 Patentblatt 86/38**

(84) Benannte Vertragsstaaten:
**CH DE FR GB IT LI NL**

(56) *Entgegenhaltungen:*
**EP - A - 0 042 820**
**DE - B - 2 157 721**
**FR - A - 2 134 582**
**FR - A - 2 466 477**

**Chemical Abstracts, Band 93, Nr. 8, 25. August 1980, Columbus, Ohio, USA G. DALL'ASTA "Effect of the type and amount of unsaturation in nylon 12,12 and its unsaturated copolymers on some physical and physicochemical properties", Seite 13, Spalte 2, Abstract Nr. 72492e**
**Chemical Abstracts, Band 68, Nr. 10, 4. März 1968, Columbus, Ohio, USA KANEGAFUCHI SPINNING LTD. "Polyamides containing double bonds", Seite 3994, Spalte 2, Abstract Nr. 40908n**

(73) Patentinhaber: **CIBA-GEIGY AG, Klybeckstrasse 141, CH-4002 Basel (CH)**

(72) Erfinder: **Berger, Joseph, Dr., Sperrstrasse 40/18, CH-4057 Basel (CH)**
Erfinder: **Pfeifer, Josef, Dr., Brunnmattstrasse 32, CH-4106 Therwil (CH)**
Erfinder: **Reinehr, Dieter, Dr., Wolfsheule 10, D-7842 Kandern (DE)**

**EP 0 087 385 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft vernetzbare transparente Polyamide, Verfahren zu deren Herstellung deren Verwendung, besonders in vernetzbaren, Polythiole enthaltenden Stoffgemischen.

Ueber C=C-Doppelbindungen vernetzbare Polyamide sind z.B. aus der japanischen Patentpublikation 9677/67, der deutschen Offenlegungsschrift 3.037.488 und Vysokomol. Soedin, Serie B, 16, 97 (1974) bekannt. Für die Herstellung dieser bekannten Polyamide werden als Diaminkomponenten unsubstituierte 1,11-Diaminoundecene, ein- oder zweifach ungesättigte unsubstituierte $\alpha,\omega$-Diamine mit 12 C-Atomen, wie das 1,12-Dodecan-5,9-cis-trans-dien-diamin, oder 1,8-Diaminooctadiene-2,6, die unsubstituiert oder durch zwei oder vier Methylgruppen substituiert sein können, verwendet. Bei diesen vorbekannten Polyamiden handelt es sich um kristalline Produkte. Nicht vernetzbare transparente Polyamide aus gesättigten, substituierten Diaminen, z.B. in 1,10-Stellung substituierten 1,10-Diaminodecanen oder 1,11-Diaminoundecanen und aromatischen oder aliphatischen Dicarbonsäuren sind z.B. in den deutschen Offenlegungsschriften 2.846.459, 2.846.501 und 2.846.514 sowie der EP Patentveröffentlichung Nr. 12712 beschrieben.

Gegenstand der Erfindung sind transparente Polyamide, die aus wiederkehrenden Strukturelementen der Formel I

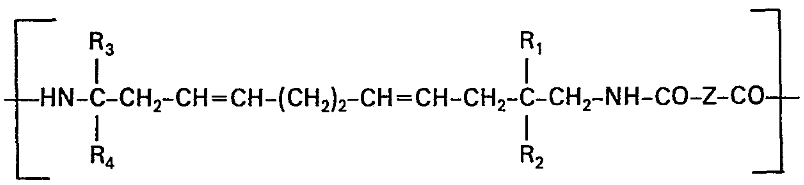 (I)

bestehen, worin

$R_1$ $C_{1-12}$-Alkyl,

$R_2$ Wasserstoff oder $C_{1-12}$-Alkyl,

$R_3$ $C_{1-12}$-Alkyl, Cycloalkyl mit 4-12 Ring-C-Atomen, Aralkyl mit 7 oder 8 C-Atomen, gegebenenfalls substituiertes Aryl, oder, wenn $R_4$ Wasserstoff ist, auch -CH=CH-Alkyl oder -C(Alkyl)=CH-Alkyl mit je 1-4 C-Atomen in den Alkylgruppen und

$R_4$ Wasserstoff, $C_{1-12}$-Alkyl, Cycloalkyl mit 4-12 Ring-C-Atomen, Aralkyl mit 7 oder 8 C-Atomen oder gegebenenfalls substituiertes Aryl darstellen oder

$R_1$ und $R_2$ und/oder $R_3$ und $R_4$ zusammen Alkylen mit 3-11 C-Atomen bedeuten und

Z in den verschiedenen Strukturelementen der Formel I gleiche oder verschiedene Reste einer gesättigten oder ungesättigten aliphatischen oder aromatischen Dicarbonsäure darstellt.

Durch $R_1$ bis $R_4$ dargestellte Alkylgruppen können geradkettig oder verzweigt sein. Alkylgruppen $R_1$, $R_2$ und $R_4$ weisen bevorzugt 1-5 C-Atome auf und sind geradkettig. Alkylgruppen $R_3$ weisen mit Vorteil 1-7 C-Atome und insbesondere 1-5 C-Atome auf. Beispiele von Alkylgruppen $R_1$ bis $R_4$ sind: die Methyl-, Äthyl-, n-Propyl-, Isopropyl-, n-, sek.- und tert.-Butyl-, n-Pentyl-, 2- oder 3-Pentyl-, n-Hexyl-, 2- oder 3-Heptyl-, n-Octyl-, n-Decyl- und n-Dodecylgruppe.

Stellt $R_3$ eine Gruppe -CH=CH-Alkyl oder -C(Alkyl)=CH-Alkyl dar, so sind die Alkylgruppen in diesen Substituenten bevorzugt geradkettig und bedeuten insbesondere Methyl oder Äthyl.

Cycloalkylgruppen $R_3$ und $R_4$ können unsubstituiert oder durch $C_{1-4}$-Alkylgruppen substituiert sein. Insbesondere handelt es sich dabei um durch eine Methyl- oder Äthylgruppe substituiertes Cycloalkyl. Bevorzugt sind Cycloalkylgruppen $R_3$ und $R_4$ jedoch unsubstituiert und weisen 5-8 Ring-C-Atome auf. Besonders bevorzugt sind die Cyclopentyl- und vor allem die Cyclohexylgruppe.

Als Aralkylgruppen $R_3$ und $R_4$ kommen vor allem die Benzyl-, Methyl-benzyl- oder Phenyläthylgruppe in Betracht. Stellt $R_3$ oder $R_4$ substituiertes Aryl dar, so kommen als Substituenten vor allem Alkylgruppen mit 1-4 und insbesondere 1 oder 2 C-Atomen in Betracht. Arylgruppen $R_3$ und $R_4$ können mehrere Alkylgruppen tragen, sind aber bevorzugt nur durch eine Alkylgruppe substituiert. Besonders bevorzugt ist die 1- oder 2-Naphthylgruppe, durch eine Alkylgruppe mit 1-4 und besonders 1 oder 2 C-Atomen substituiertes Phenyl und ganz besonders unsubstituiertes Phenyl.

Durch $R_1$ und $R_2$ und/oder $R_3$ und $R_4$ zusammen dargestellte Alkylengruppen weisen bevorzugt 4-7 C-Atome auf. Insbesondere handelt es sich dabei um die Tetramethylen- und ganz besonders die Pentamethylengruppe.

Die erfindungsgemässen Polyamide können auf an sich bekannte Weise dadurch hergestellt werden, dass man ein Diamin der Formel II

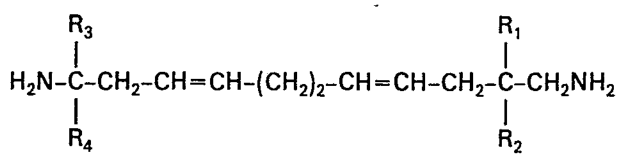 (II)

mit einer Dicarbonsäure der Formel III

HOOC-Z-COOH (III),

einem amidbildenden Derivat davon oder einem Gemisch verschiedener Dicarbonsäuren der Formel III bzw. amidbildender Derivate davon umsetzt. Dabei haben $R_1$ bis $R_4$ und Z die oben angegebene Bedeutung.

Als amidbildende Derivate der Dicarbonsäuren der Formel III können beispielsweise die entsprechenden Dihalogenide, vor allem die Dichloride, Dinitrile, Dialkyl- oder Diarylester, besonders Diphenylester und Dialkylester mit je 1–4 C-Atomen in den Alkylteilen verwendet werden.

Im allgemeinen ist es von Vorteil, die Polykondensation in Gegenwart einer Phosphorverbindung als Beschleuniger durchzuführen. Dadurch werden kürzere Reaktionszeiten erzielt, und die Polykondensation kann bei hohen Temperaturen, beispielsweise in der Schmelze, vorgenommen werden. Gegenstand der Erfindung ist daher auch ein Verfahren zur Herstellung transparenter Polyamide, dadurch gekennzeichnet, dass man wie oben beschrieben ein Diamin der Formel II mit einer Dicarbonsäure der Formel III, einem amidbildenden Derivat davon oder einem Gemisch verschiedener Dicarbonsäuren der Formel III oder amidbildender Derivate davon in Gegenwart einer 30 bis 1000 ppm, vorzugsweise 80 bis 500 ppm Phosphor, bezogen auf die Summe der Kondensationskomponenten, enthaltenden Phosphorverbindung polykondensiert.

Als Phosphorverbindungen können sowohl anorganische als auch organische Verbindungen verwendet werden, wie Sauerstoffsäuren des Phosphors und Derivate davon, z.B. Ester oder Amide, oder Phosphin- und Phosphonsäuren und Derivate davon.

Als anorganische Phosphorverbindungen kommen z.B. Phosphorsäure, phosphorige und hypophosphorige Säure und Derivate davon in Betracht, wie mono-Ammoniumphosphate, di-Ammoniumhydrogenphosphate, $NH_{4H2}PO_3$ und $NH_4H_2PO_2$, Ester und Amide, vor allem Alkylester mit je 1–8 C-Atomen in den Alkylgruppen und Phenylester, oder Dialkylamide mit je 1–4 C-Atomen in den Alkylgruppen. Als Beispiele solcher Derivate seien genannt: Diäthylphosphit, Dibutylphosphit, Triphenylphosphit, Trimethylphosphat, Triäthylphosphat, Tributylphosphat, Triisobutylphosphat, Triphenylphosphat, Trikresylphosphat, Hexamethylphosphorsäuretriamid. Geeignet sind ferner auch Pyrophosphorsäure und Polyphosphorsäuren.

Geeignete organische Phosphorverbindungen sind z.B. Mono- und Polyphosphonsäuren, Phosphinsäuren und deren Derivate. Als Beispiele von Polyphosphonsäuren können

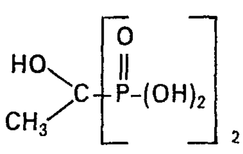

und

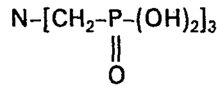

genannt werden.

Insbesondere eignen sich Monophosphonsäuren und Monophosphinsäuren der Formeln IVa und IVb

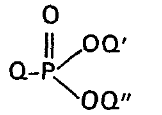 (IVa) und 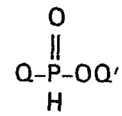 (IVb),

worin Q gegebenenfalls substituiertes Aryl, Aralkyl oder Alkyl darstellt und Q′ und Q″ unabhängig voneinander Wasserstoff darstellen oder die gleiche Bedeutung haben können wie Q, bevorzugt aber Wasserstoff oder $C_{1\text{-}4}$-Alkyl darstellen. Bevorzugt sind Verbindungen der Formeln IVa und IVb, worin Q Phenyl oder

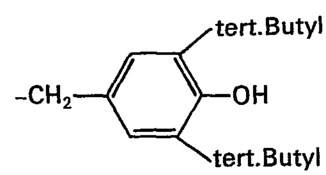

bedeutet.

Besonders bevorzugt verwendet man als organische Phosphorverbindung 2,6-Di-tert.-butyl-p-kresylphosphonsäurediäthylester oder als anorganische Phosphorverbindungen Phosphorsäure oder $NH_4H_2PO_2$.

Vorzugsweise werden die erfindungsgemässen Polyamide nach dem Schmelzpolykondensationsverfahren in mehreren Stufen hergestellt. Dabei werden die Reaktionskomponenten in im wesentlichen stöchiometrischen Mengen, bevorzugt Salze aus einer Dicarbonsäure der Formel III und einem Diamin der Formel II, unter Druck bei Temperaturen zwischen etwa 220 und 300°C in der Schmelze unter Inertgas, wie Stickstoff, vorkondensiert. Das Vorkondensat kann anschliessend bei Temperaturen zwischen etwa 220 und 300°C bei Normaldruck und zweckmässig ebenfalls in Inertgasatmosphäre bis zur Bildung der erfindungsgemässen Polyamide weiterkondensiert werden. Unter Umständen kann es von Vorteil sein, nach Beendigung der Polykondensation Vakuum anzulegen, um das Polyamid zu entgasen. Dieses Verfahren eignet sich besonders zur Herstellung von Polyamiden unter Verwendung von aliphatischen Dicarbonsäuren mit 6–36 C-Atomen und/oder aromatischen Dicarbonsäuren.

Die erfindungsgemässen Polyamide können auch durch Schmelzpolykondensation von Diaminen der Formel II mit im wesentlichen stöchiometrischen Mengen eines aktivierten Esters einer Dicarbonsäure der Formel III hergestellt werden. Als aktivierte Ester eignen sich vor allem die entsprechenden Diphenylester. Die Reaktionstem-

peraturen liegen dabei im allgemeinen zwischen etwa 230 und 300°C. Dieses Verfahren ist besonders bevorzugt, wenn $R_4$ $C_{1-12}$-Alkyl oder $R_3$ und $R_4$ zusammen $C_{3-11}$-Alkylen darstellen.

Schliesslich können die erfindungsgemässen Polyamide auch in an sich bekannter Weise durch Lösungsmittelpolykondensation, bevorzugt unter Verwendung von Säurehalogeniden und in Gegenwart von säurebindenden Mitteln, oder durch Grenzflächenpolykondensation hergestellt werden. Diese Verfahren werden bevorzugt, wenn aliphatische Dicarbonsäuren mit 4 oder 5 C-Atomen eingesetzt werden.

Im allgemeinen ist es zweckmässig, bei den Polykondensationsreaktionen zur Verhinderung einer frühzeitigen Vernetzung Polymerisationsinhibitoren mitzuverwenden, z.B. Hydrochinon oder sterisch gehinderte Phenole, wie Di-tert.-butyl-p-kresol.

Bevorzugt sind Polyamide, worin $R_1$ $C_{1-5}$-Alkyl, $R_2$ Wasserstoff oder $C_{1-5}$-Alkyl oder $R_1$ und $R_2$ zusammen Alkylen mit 4–7 C-Atomen, $R_3$ $C_{1-7}$-Alkyl, $C_{5-8}$-Cycloalkyl oder unsubstituiertes Phenyl oder, bei $R_4$=H, auch $-C(C_2H_5)=CH-CH_3$ und $R_4$ Wasserstoff oder $C_{1-5}$-Alkyl bedeuten und Z den Rest der Terephthalsäure, Isophthalsäure, einer gesättigten aliphatischen Dicarbonsäure mit 6–12 C-Atomen und/oder einer ungesättigten aliphatischen Dicarbonsäure mit 6–36 C-Atomen bedeutet. Besonders bevorzugt sind Polyamide, worin $R_1$ $C_{1-5}$-Alkyl, $R_2$ Wasserstoff oder $C_{1-5}$-Alkyl oder $R_1$ und $R_2$ zusammen Alkylen mit 4–7 C-Atomen, $R_3$ $C_{1-5}$-Alkyl, unsubstituiertes Phenyl oder, bei $R_4$=H, $-C(C_2H_5)=CHCH_3$ und $R_4$ Wasserstoff oder Methyl bedeuten und Z den Rest der Terephthalsäure, Isophthalsäure, einer gesättigten aliphatischen Dicarbonsäure mit 6–12 C-Atomen und/oder einer ungesättigten aliphatischen Dicarbonsäure mit 36 C-Atomen (Dimersäure) darstellt, und vor allem Polyamide, worin $R_1$ Methyl oder Äthyl, $R_2$ Wasserstoff, Methyl oder Äthyl, $R_3$ $C_{1-5}$-Alkyl oder unsubstituiertes Phenyl und $R_4$ Wasserstoff oder Methyl bedeuten und Z den Rest der Terephthalsäure, Isophthalsäure und/oder einer gesättigten aliphatischen Dicarbonsäure mit 6–10 C-Atomen darstellt.

Unter den Homopolyamiden werden ganz besonders diejenigen bevorzugt, worin $R_1$ und $R_2$ Methyl, $R_3$ Isopropyl, $R_4$ Wasserstoff und Z den Rest der Terephthalsäure, Isophthalsäure oder Adipinsäure bedeuten.

Ganz besonders bevorzugt sind jedoch Copolyamide, worin $R_1$ und $R_2$ Methyl, $R_3$ Isoproypl, $R_4$ Wasserstoff und Z in 50 bis 90%, besonders 60 bis 85%, der Strukturelemente der Formel I den Rest der Terephthalsäure und in 50 bis 10%, besonders 45 bis 15%, der Strukturelemente der Formel I den Rest der Adipinsäure darstellen bzw. in Gegenwart einer 80 bis 500 ppm Phosphor enthaltenden Phosphorverbindung, vor allem $H_3PO_4$, $NH_4H_2PO_2$ oder 2,6-Di-tert.-butyl-p-kresylphosphonsäurediäthylester, erhältliche Polyamide, worin $R_1$ und $R_2$ Methyl, $R_3$ Isopropyl, $R_4$ Wasserstoff und Z in 50 bis 90 Gew.-%, besonders 60 bis 85 Gew.-% der Verbindungen der Formel III den Rest der Terephthalsäure und in 50 bis 10 Gew.-%, besonders 40 bis 15 Gew.-% der Verbindungen der Formel III den Rest der Adipinsäure darstellen, wobei sich die Gewichtsprozente auf gleiche funktionelle Gruppen der Dicarbonsäuren beziehen.

Die Dicarbonsäuren der Formel III bzw. deren amidbildende Derivate sind bekannt. Die Diamine der Formel II können dadurch hergestellt werden, dass man Verbindungen der Formel IIa

$$H_2N-\underset{R_4}{\overset{R_3}{C}}-CH_2-CH=CH-(CH_2)_2-CH=CH-CH_2-\underset{R_2}{\overset{R_1}{C}}-CH=NOH \qquad (IIa)$$

entweder direkt zu Verbindungen der Formel II reduziert oder die Verbindungen der Formel IIa zuerst zu Verbindungen der Formel IIb

$$H_2N-\underset{R_4}{\overset{R_3}{C}}-CH_2-CH=CH-(CH_2)_2-CH=CH-CH_2-\underset{R_2}{\overset{R_1}{C}}-C\equiv N \qquad (IIb)$$

dehydratisiert und anschliessend die Verbindungen der Formel IIb zu Verbindungen der Formel II reduziert, wobei $R_1$ und $R_4$ die unter Formel I angegebene Bedeutung haben.

Die Reduktion der Verbindungen der Formel IIa oder IIb zu Verbindungen der Formel II kann in an sich bekannter Weise erfolgen, z.B. mittels Bouveault-Blanc-Reduktion mit Natriummetall und Alkoholen, gegebenenfalls in Gegenwart eines inerten organischen Lösungsmittels, z.B. aromatischen Kohlenwasserstoffen, wie Benzol, Toluol oder Xylol.

Die allfällige Dehydratisierung der Verbindungen der Formel IIa zu Verbindungen der Formel IIb kann ebenfalls in an sich bekannter Weise chemisch oder thermisch durchgeführt werden. Die Ausgangsprodukte der Formel IIa sind bekannt und können nach dem in der europäischen Patentschrift 11599 beschriebenen Verfahren hergestellt werden.

Die erfindungsgemässen Polyamide eignen sich beispielsweise zum Erzeugen von lösungsmittelbeständigen Überzügen auf verschiedenen Materialien, vor allem Metallen, wie Aluminium,

Kupfer und Stahl, zur Bilderzeugung unter Lichteinwirkung auf verschiedenen anorganischen oder organischen Substraten oder zur Herstellung von transparenten Formkörpern z.B. nach dem Spritzguss- oder Extrusionsverfahren. Geeignete Substrate für die Bilderzeugung sind z.B. Glas, Metalle und Metalloxide, wie Aluminium, Aluminiumoxid und Kupfer, Keramik, Papier und hochmolekulare organische Materialien. Als hochmolekulare organische Materialien kommen natürliche und synthetische Polymere in Betracht, z.B. Cellulosematerialien, wie Celluloseacetate, Cellulosepropionate, Cellulosebutyrate und Celluloseäther; vernetzte Epoxidharze, Polyacetale, Polyphenylenoxide, gesättigte Polyamide, Polyolefine, wie Polyäthylen und Polypropylen und Copolymere davon; Vinylpolymere, wie Polyvinylchlorid, Polyvinylacetat, Polyvinylalkohol und Copolymere davon, und vor allem Polyester.

Die erfindungsgemässen Polyamide können leicht thermisch oder photochemisch vernetzt werden, gegebenenfalls unter Zusatz an sich bekannter radikalischer Initiatoren. Die thermische Vernetzung erfolgt im allgemeinen bei Temperaturen zwischen 80 und 180°C, besonders 120 und 160°C. Die radikalischen Initiatoren werden zweckmässig in Mengen von 0,01 bis 5 Gew.-%, vorzugsweise 0,01 bis 1,5 Gew.-%, bezogen auf das Gesamtgewicht des Polyamids, verwendet.

Geeignete Initiatoren sind z.B. anorganische oder organische Peroxide oder Azoverbindungen, z.B. Wasserstoffperoxid, Kaliumperoxidisulfat, tert.-Butylhydroperoxid, Di-tert.-butylperoxid, Peressigsäure, Dibenzoylperoxid, Diacylperoxide, Cumolhydroperoxid, tert.-Butylperbenzoat, ter.-Alkylperoxidicarbonate und $\alpha,\alpha'$-Azoisobutyronitril.

Die photochemische Vernetzung wird vorzugsweise in Gegenwart von Polythiolen und Photoinitiatoren vorgenommen.

Gegenstand der Erfindung sind deshalb auch vernetzbare Stoffgemische, enthaltend

A) ein erfindungsgemässes Polyamid,

B) 5 bis 80 Gew.-%, besonders 30 bis 65 Gew.-%, bezogen auf das Polyamid, eines Polythiols mit mindestens zwei Thiolgruppen pro Molekül und

C) 0,1 bis 10 Gew.-%, besonders 1 bis 5 Gew.-%, bezogen auf das Polyamid, eines unter Lichteinwirkung radikalisch spaltbaren Initiators oder eines Photosensibilisators. Dabei gilt in bezug auf bevorzugte Bedeutungen von $R_1$ bis $R_4$ das unter Formel I Angegebene.

Beispiele geeigneter Photosensibilisatoren C) sind Benzophenon, Acetophenon, 1,4-Diacetylbenzol, Xanthon, Thioxanthone, 2-Acetonaphthen, 1-Acetonaphthol, Michlers Keton, Thiophen, Benzanthron und Benzoinäther. Als Initiatoren können radikalische Initiatoren der oben erwähnten Art eingesetzt werden. Bevorzugt wird als Photosensibilisator Benzophenon oder Thioxanthon verwendet.

Geeignete Polythiole sind z.B. in der US-PS 3.824.104 beschrieben. Bevorzugt sind Verbindungen der Formeln V und VI

$Q_1$-$(SH)_n$ (V) und $Q_1$-$(OCO$-$Q_2$-$SH)_{n_1}$ (VI).

Darin bedeuten n und $n_1$ eine ganze Zahl von mindestens 2, $Q_1$ ist ein n- bzw. $n_1$-wertiger, keine C-C-Mehrfachbindungen aufweisender organischer Rest, der auch Heteroatome enthalten kann, und $Q_2$ ist ein zweiwertiger, keine C-C-Mehrfachbindungen aufweisender Rest.

n ist vorzugsweise 2 und $Q_1$ in Formel V bedeutet insbesondere Phenylen, Tolylen, Xylylen oder $-C_mH_{2m}-$ mit m = 2–12. Als Beispiele von Verbindungen der Formel V seien genannt: Phenylen-1,4-dithiol, Tolylen-2,4-dithiol, m-Xylylen-2,5-dithiol, Äthandithiol, Tetramethylendithiol, Hexamethylendithiol und Decamethylendithiol.

$n_1$ ist bevorzugt 2, 3 oder 4. $Q_2$ stellt insbesondere $-C_pH_{2p}-$ mit p = 1–3 dar, besonders $-CH_2$, $-CH_2CH_2-$ oder $-CH(CH_3)-$. $Q_1$ in Formel VI bedeutet vorzugsweise einen Rest $-C_mH_{2m}-$, vor allem $-CH_2CH_2-$, $-CH_2\overset{|}{C}HCH_2-$ oder $C(CH_2-)_4$. Beispiele von Verbindungen der Formel VI sind Äthylenglykol-bis-(thioglykolat), Äthylenglykol-bis-(3-mercaptopropionat), Trimethylolpropan-tris-(thioglykolat), Trimethylolpropan-tris-(3-mercaptopropionat), Pentaerythritol-tetrakis-(thioglykolat) und Pentaerythritol-tetrakis-(3-mercaptopropionat). Bei den Verbindungen der Formeln V und VI kann es sich auch um Polymere handeln, wie Polypropylenglykol-bis-(3-mercaptopropionat). Geeignet sind auch niedermolekulare Thioplaste mit Mercaptanendgruppen, die im allgemeinen als Gemische mit verschiedenen organischen Resten vorliegen, wie z.B. Polymere von Bis-(äthylenoxy)methan mit Disulfid-Brücken der Thiokol Chemical Corp. (vgl. z.B. US Patentschriften 2.402.977 und 2.875.182). Bevorzugt sind Verbindungen der Formel VI, worin $Q_1$, $Q_2$ und $n_1$ die oben angegebenen bevorzugten Bedeutungen haben, ganz besonders Pentaerythritol-tetrakis-(3-mercaptopropionat).

Derartige vernetzbare Stoffzusammensetzungen können auch weitere an sich bekannte Zusätze, wie Stabilisatoren, Antioxidantien, Farbstoffe, Pigmente, antistatische oder flammhemmende Mittel, Weichmacher und Füllstoffe enthalten.

Die genannten Polythiole enthaltenden Stoffgemische eignen sich vor allem zur Herstellung von gedruckten Schaltungen.

Für die photochemische Vernetzung können an sich beliebige geeignete Lichtquellen eingesetzt werden, z.B. Xenonlampen, Metallhalogenidlampen und besonders Quecksilberhochdruck- und mitteldrucklampen.

Die erfindungsgemässen transparenten Polyamide zeichnen sich durch geringe Wasseraufnahme, hohe Hydrolysebeständigkeit und/oder gute Dimensionsstabilität unter Feuchtigkeitseinwirkung aus. Sie ergeben ferner sehr gut haftende, lösungsmittelbeständige Überzüge, besonders auf Metallen, und weisen – im Gegenstz zu vorbekannten ungesättigten Polyamiden – eine gute Verträglichkeit mit Polythiolen auf. Dank ihrer guten Löslichkeit in verschiedenen organischen Lösungsmitteln, wie Chloroform, Äthanol und N,N-Dimethylformamid, lassen sich leicht Filme und Beschichtungen auf Metall- oder Kunststoffoberflächen herstellen.

Beispiel 1

62,8 g 2,2-Dimethyl-11-isopropyl-1,11-diamino-undeca-4,8,dien, 36,4 g Adipinsäure, 0,25 ml einer 10%igen wässrigen $NH_4H_2PO_2$-Lösung und 0,5 g Di-tert.-butyl-p-kresol werden in einem Autoklaven 90 Minuten in Stickstoffatmosphäre vorkondensiert, dann 4 Stunden in einem offenen Polykondensationsgefäss im Stickstoffstrom weiterkondensiert und schliesslich 1 Stunde im Hochvakuum nachkondensiert. Alle Polykondensationsschritte werden bei 250°C durchgeführt. Elementaranalyse des erhaltenen Polyamids:

| | | | |
|---|---|---|---|
| Berechnet: | C 72,88%; | H 10,57%; | N 7,73% |
| Gefunden: | C 70,20%; | H 10,47%; | N 7,45%. |

Endgruppengehalt: -COOH 0,16 m Äquiv./g; $-NH_2$ 0,04 m Äquiv./g. Glasumwandlungstemperatur (Tg, bestimmt im Differentialscanningkalorimeter) = 45°C; reduzierte Viskosität $\eta_{red.}$ = 0,70 dl/g (gemessen als 0,5%ige Lösung in m-Kresol bei 25°C).

Das verwendete Diamin kann wie folgt hergestellt werden:

80 g (0,3 Mol) 2,2-Dimethyl-11-isopropyl-11-amino-undeca-4,8-dienaloxim werden unter Rühren mit 100 ml Eisessig versetzt. Dann wird HCl-Gas bis zur Sättigung eingeleitet, und innerhalb von 15 Minuten werden 30,6 g (0,3 Mol) Essigsäureanhydrid zugetropft. Man erhitzt die Reaktionsmischung noch 4 Stunden unter Rückfluss, destilliert den Eisessig ab und löst den Rückstand in Wasser. Nachdem man die Lösung mit Natronlauge basisch gestellt hat, nimmt man die sich abscheidende organische Phase mit Toluol auf und destilliert. Man erhält 68,5 g (0,276 Mol) 1,1-Dimethyl-10-isopropyl-10-amino-deca-3,7-dien-nitril, entsprechend einer Ausbeute von 92% d.Th.; Sdp. 94°C/3 Pa.

23 g (1 Mol) Natrium werden zu 150 ml Toluol gegeben, und die Mischung wird bis zum Schmelzen des Natriums aufgeheizt. Dann wird die Heizung entfernt und solange gerührt, bis das Natrium als feingraue Dispersion zerteilt ist. Zu dieser Mischung tropft man dann eine Lösung von 53 g (0,214 Mol) 1,1-Dimethyl-10-isopropyl-10-amino-deca-3,7-dien-nitril in 100 ml Isopropanol. Es wird noch 3 Stunden unter Rückfluss gekocht, mit 200 ml Wasser versetzt, und die organische Phase wird abgetrennt. Nach dem Abdestillieren des Lösungsmittels erhält man 44 g (0,175 Mol) 2,2-Dimethyl-11-isopropyl-1,11-diamino-undeca-4,8-dien, entsprechend einer Ausbeute von 81,5% d.Th.; Sdp. 86°C/1 Pa; $n_D^{20}$ = 1,4810.

Beispiele 2a und 2b

Auf die in Beispiel 1 beschriebene Weise werden 38,21 g Terephthalsäure mit 58,06 g 2,2-Dimethyl-11-isopropyl-1,11-diamino-undeca-4,8-dien bzw. 29,26 g Isophthalsäure mit 46,0 g 2,2-Dimethyl-11-isopropyl-1,11-diamino-undeca-4,8-dien in Gegenwart von 0,25 ml einer 10%igen wässrigen $NH_4H_2PO_2$-Lösung und 0,5 g Di-tert.-butyl-p-kresol polykondensiert. Die Eigenschaften des erhaltenen Polyamids sind in der folgenden Tabelle angegeben.

Beispiel 3

Herstellung eines Salzes aus Terephthalsäure (TPS) und 2,2-Dimehtyl-11-isopropyl-1,11-diamino-undeca-4,8-dien (Undecadiendiamin = UDD):

97,1 g Terephthalsäure werden in 2400 ml Wasser und 600 ml Methanol suspendiert. Zur erhaltenen Suspension tropft man bei Rückflusstemperatur 148,9 g UDD zu, wobei eine homogene Lösung entsteht. Nach 60 Minuten Kochen lässt man die Lösung auf 0–5°C abkühlen. Nach 24 Stunden wird vom gebildeten Salz abfiltriert und bei 80°C im Vakuum getrocknet. Ausbeute 180 g (73,6% d.Th.).

60 g des so erhaltenen Salzes werden wie in Beispiel 1 beschrieben polykondensiert. Die Eigenschaften des erhaltenen Polyamids sind in der folgenden Tabelle angegeben.

Beispiel 4

30 g gemäss Beispiel 3 erhaltenen Salzes aus TPS und UDD werden wie in Beispiel 1 beschrieben polykondensiert, jedoch unter Weglassen der letzten Kondensationsstufe im Vakuum. Die Eigenschaften des erhaltenen Polyamids sind in der folgenden Tabelle angegeben.

Beispiel 5

Herstellung eines Salzes aus Adipinsäure (AS) und UDD: 36,2 g Adipinsäure werden in 270 ml absolutem Äthanol bei 50°C gelöst. Nach dem Abkühlen der Lösung werden 69,7 g UDD in 107 ml absolutem Äthanol zugegeben. Das Salz, das nach Abziehen von zwei Dritteln des Lösungsmittels und Abkühlen des Restes auf 0–5°C ausfällt, wird abfiltriert und im Vakuum bei 20°C getrocknet. Ausbeute 82,5 g (85,3% d.Th.).

56,0 g des gemäss 3 erhaltenen Salzes aus TPS und UDD sowie 14,0 g des obigen Salzes aus Adipinsäure und UDD werden wie in Beispiel 1 beschrieben polykondensiert, jedoch unter Weglassen der letzten Kondensationsstufe im Vakuum. Die Eigenschaften des erhaltenen Polyamids sind in der folgenden Tabelle angegeben.

Beispiel 6

30,0 g des gemäss Beispiel 3 hergestellten Salzes aus TPS und UDD sowie 30,0 g des gemäss Beispiel 5 hergestellten Salzes aus Adipinsäure und UDD werden wie in Beispiel 1 beschrieben polykondensiert, jedoch unter Weglassen der Polykondensationsstufe im Vakuum. Die Eigenschaften des erhaltenen Polyamids sind in der folgenden Tabelle angegeben.

Beispiel 7

Beispiel 6 wird wiederholt, jedoch unter Verwendung von 54,0 g des Salzes aus TPS und UDD und 6,0 g des Salzes aus Adipinsäure und UDD. Die Eigenschaften des erhaltenen Polyamids sind in der folgenden Tabelle angegeben.

Die Polykondensationen gemäss den Beispielen 3-7 werden alle in Gegenwart von 0,25 ml einer 10%igen wässrigen $NH_4H_2PO_2$-Lösung und 0,5 g Di-tert.-butyl-p-kresol durchgeführt.

Beispiel 8

50,0 g UDD, 28,98 g Adipinsäure, 79 mg der Verbindung der Formel

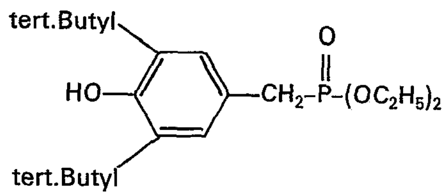

und 0,4 g Di-tert.-butyl-p-kresol werden wie in Beispiel 1 beschrieben polykondensiert.

Elementaranalyse des erhaltenen Polyamids:

Berechnet: C 72,88%; H 10,57%; N 7,79%
Gefunden: C 71,20%; H 10,51%; N 7,68%.

Endgruppengehalt -COOH 0,15 m/Äquiv./g, $-NH_2$ 0,05 m/Äquiv./g. Die übrigen Eigenschaften des erhaltenen Polyamids sind in der folgenden Tabelle I angegeben.

Tabelle 1

| Polyamid gemäss Beispiel Nr. | Kondensationskomponenten | Glasumwandlungs-temperatur Tg (°C) **) | reduzierte Viskosität η red *) dl/g |
|---|---|---|---|
| 2a | 38,21 g TPS, 58,06 g UDD | 108 | 0,23 |
| 2b | 29,26 g IPS, 46,0 g UDD | 100 | 0,34 |
| 3 | 60,0 g TPS/UDD-Salz | 114 | nicht löslich |
| 4 | 30,0 g TPS/UDD-Salz | 115 | 0,78 |
| 5 | 56,0 g TPS/UDD-Salz<br>14,0 g AS/UDD-Salz | 105 | 0,72 |
| 6 | 30,0 g TPS/UDD-Salz<br>30,0 g AS/UDD-Salz | 73 | 0,75 |
| 7 | 54,0 g TPS/UDD-Salz<br>6,0 g AS/UDD-Salz | 101 | 0,78 |
| 8 | 28,98 g AS<br>50,0 g UDD | 42 | 0,68 |

TPS = Terephthalsäure
IPS = Isophthalsäure
AS = Adipinsäure
UDD = 2,2-Dimethyl-11-isopropyl-1,11-diamino-undeca-4,8-dien
*) gemessen als 0,5%ige Lösung in m-Kresol bei 25°C
**) bestimmt im Differentialscanningkalorimeter (DSC)

Beispiel 9

7,03 g 2,2-Dimethyl-11-isopropyl-1,11-diamino-undeca-4,8-dien von einer 92%igen Reinheit (0,026 Mol) und 21,0 g dimerisierte Ölsäure ($C_{36}H_{68}O_4$; 0,037 Mol) werden in einem Autoklaven während 120 Minuten bei 200-250°C vorkondensiert, dann bei 250°C während 120 Minuten im Stickstoffstrom weiterkondensiert und schliesslich 1 Stunde lang im Hochvakuum nachkondensiert.

Polymer-Tg = -19°C, $\eta_{red.}$ = 0,12 dl/g (0,5%ige Lösung in m-Kresol bei 25°C).

Beispiel 10

15,82 g 2,2-Dimethyl-11-isopropyl-1,11-diamino-undecan-4,8-dien (Reinheit 99,5%; 0,0024 Mol), 18,86 g dimerisierte Ölsäure ($C_{36}H_{68}O_4$; 0,0334 Mol), 4,28 g Adipinsäure (0,0293 Mol), 0,1 ml einer 10%igen wässrigen $NH_4H_2PO_2$-Lösung und 0,2 g Di-tert.butyl-p-kresol werden in einem

Autoklaven während 90 Minuten bei 250°C in Stickstoffatmosphäre kondensiert.

Polymer-Tg = +14°C, $\eta_{red.}$ = 0,24 dl/g (0,5%ige m-Kresol-Lösung bei 25°C).

Beispiel 11

Herstellung des Polyamids aus 1,2,3,6-Tetrahydrophthalsäurechlorid (THPC) und 2,2 -Dimethyl-11 -isopropyl- 1,11 -diaminoundeca- 4,8 -dien (UDD).

Zu einer Lösung aus 9,37 g UDD (Reinheitsgrad 97,5 %) und 7,33 g Triäthylamin in 315 g $CH_2Cl_2$, die auf -15°C abgekühlt wurde, wird eine Lösung von 7,5 g THPC in 100 g $CH_2Cl_2$ während 1 Stunde zugetropft. Die Lösung wird während weiteren 90 Minuten bei -15°C und 15 Stunden bei +20°C gerührt. Das Lösungsmittel wird abgezogen, der Rückstand in 50 ml Äthanol gelöst und das Polymere in 4 l $H_2O$ gefällt, filtriert, gewaschen und im Hochvakuum bei 20°C getrocknet. Ausbeute 13,7 g (97,9 % d.Th.), $\eta_{red.}$ = 0,13 dl/g (0,345%ige Lösung in m-Kresol bei 25°C),

Elementaranalyse:
C 73,77% (theor. 74,57%), H 9,94% (theor. 9,91%), N 7,14% (theor. 7,25%).

Tg = 60°C.

Herstellung des 1,2,3,6-Tetrahydrophthalsäurechlorids

17,4 g 1,2,3,6-Tetrahydrophthalsäure (0,1023 Mol) und 42,5 g Phosphorpentachlorid (0,2040 Mol) werden in ein Einschlussrohr gegeben. Das Rohr wird dicht verschlossen und bei 20°C geschüttelt, bis sich der Inhalt verflüssigt. Nachdem der Überdruck beseitigt ist, wird das Rohr auf 90°C aufgeheizt und der Inhalt bei dieser Temperatur während 3 Stunden reagieren gelassen. Das Produkt wird durch Destillation bei 17.33.10² Pa/133-135°C isoliert. Ausbeute: 13,0 g (61,4% d.Th.)

Elementaranalyse:

C 47,21% (theor. 46,41%), H 4,511 (theor. 3.89%), Cl 33,26% (theor. 34,24%).

Beispiele 12 bis 14

Polyamide aus Adipinsäure und Diamin der folgenden Formel:

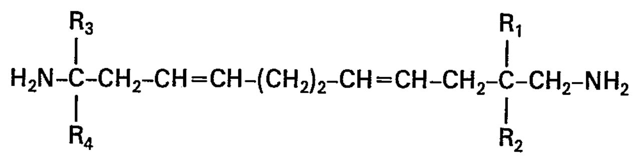

Die Substituenten $R_1$-$R_4$ haben die in der Tabelle II angegebenen Bedeutungen. Die Polyamide werden hergestellt wie im Beispiel 1 beschrieben ist. Die Reaktionszeiten sowie Angaben über $\eta_{red.}$ und Tg der Polyamide sind in der Tabelle II angeführt.

Tabelle II

| Beispiel | $R_1$ | $R_2$ | $R_3$ | $R_4$ | Diamin-reinheit (%) | Polyamid | |
|---|---|---|---|---|---|---|---|
| | | | | | | η red (dl/g) | Tg (°C) |
| 12 | $CH_3$ | H | $CH_3$ | $CH_3$ | 96 | 0,32 | +35 |
| 13 | $CH_2$-$CH_3$ | $CH_2$-$CH_3$ | $CH(C_2H_5)_2$ | H | 95,4 | 0,31 | +54 |
| 14 | $CH_3$ | $CH_3$ | $CH_2$-$CH_3$ | H | 98,2 | 0,51 | +56 |

Anwendungsbeispiele

I. Eine 10%ige Lösung des gemäss Beispiel 1 hergestellten Polyamids in Chloroform/Äthanol (1:1), die 5 Gew.% Cumoylhydroperoxid enthält, wird mit Hilfe eines 50 µm-Rakels auf eine Kupfer-Leiterplatte gegossen. Die Beschichtung wird 3 Minuten bei 100°C getrocknet, worauf die Schichtdicke ca. 5 µm beträgt. Eine Nachhärtung bei 150°C während 90 Minuten in Stickstoffatmosphäre ergibt eine harte, transparente und glänzende Schicht mit guter Haftung auf dem Kupfer. Lässt man die so behandelte Platte 72 Stunden in 60°C warmem Chloroform stehen, so wird das Polyamid nicht abgelöst, und die Haftung des Polyamids reicht aus, um eine Ätzung des darunterliegenden Kupfers zu verhindern. Nach einer Woche Lagerung in Wasser bei 20°C behält die Beschichtung ihre Transparenz und gute Haftung auf dem Kupfer; Wasseraufnahme unter 1 Gew.%.

II. a) 2,5 g des gemäss Beispiel 1 erhaltenen Polyamids, 1,5 g Pentaerythritol-tetrakis-(3-mercaptopropionat) und 95 mg Thioxanthon werden in 10,8 ml Chloroform gelöst und mit einem 50 µm-Rakel auf eine 100 µm-dicke Polyesterfolie aufgetragen. Die Beschichtung wird 3 Minuten bei 100°C getrocknet und mit einem 5000 W Quecksilberhochdruckbrenner durch eine photographische Maske während 15 Sekunden belichtet (Abstand des Quecksilberhochdruckbrenners vom Vakuumtisch 70 cm). Nach 30 Sekunden Entwicklung in Chloroform erhält man ein gut aufgelöstes Negativ-Reliefbild.

b) Das unter a) beschriebene Vorgehen wird

wiederholt unter Verwendung von 81,6 mg Benzophenon anstelle des Thioxanthons und einer Belichtungszeit von 60 Sekunden.

| Versuch | Belichtungszeit (Sekunden) | letzte abgebildete Stufe auf einem 21 stepsensitivity guide der Fa. Stouffer |
|---|---|---|
| a | 15 | 8 |
| b | 60 | 8 |

**Patentansprüche**

1. Transparente Polyamide, die aus wiederkehrenden Strukturelementen der Formel I

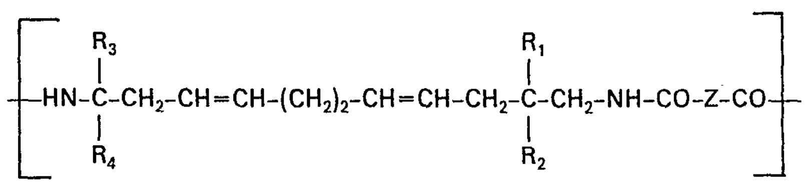 (I)

bestehen, worin
$R_1$ $C_{1-12}$-Alkyl,
$R_2$ Wasserstoff oder $C_{1-12}$-Alkyl,
$R_3$ $C_{1-12}$-Alkyl, Cycloalkyl mit 4–12 Ring-C-Atomen, Aralkyl mit 7 oder 8 C-Atomen, gegebenenfalls substituiertes Aryl, oder, wenn $R_4$ Wasserstoff ist, auch -CH=CH-Alkyl oder -C(Alkyl)=CH-Alkyl mit je 1–4 C-Atomen in den Alkylgruppen und
$R_4$ Wasserstoff, $C_{1-12}$-Alkyl, Cycloalkyl mit 4–12 Ring-C-Atomen, Aralkyl mit 7 oder 8 C-Atomen oder gegebenenfalls substituiertes Aryl darstellen oder
$R_1$ und $R_2$ und/oder $R_3$ und $R_4$ zusammen Alkylen mit 3–11 C-Atomen bedeuten und
Z in den verschiedenen Strukturelementen der Formel I gleiche oder verschiedene Reste einer gesättigten oder ungesättigten aliphatischen oder aromatischen Dicarbonsäure darstellt.

2. Polyamide nach Anspruch 1, worin $R_1$ $C_{1-5}$-Alkyl, $R_2$ Wasserstoff oder $C_{1-5}$-Alkyl < der $R_1$ und $R_2$ zusammen Alkylen mit 4–7 C-Atomen, $R_3$ $C_{1-7}$-Alkyl, $C_{5-8}$-Cycloalkyl oder unsubstituiertes Phenyl oder, bei $R_4$ = H, auch -$C(C_2H_5)$=CH-$CH_3$ und $R_4$ Wasserstoff oder $C_{1-5}$-Alkyl bedeuten und Z den Rest der Terephthalsäure, Isophthalsäure, einer gesättigten aliphatischen Dicarbonsäure mit 6–12 C-Atomen und/oder einer ungesättigten aliphatischen Dicarbonsäure mit 6–36 C-Atomen bedeutet.

3. Polyamide nach Anspruch 2, worin $R_3$ $C_{1-5}$-Alkyl, unsubstituiertes Phenyl oder, bei $R_4$ = H, -$C(C_2H_5)$=CH-$CH_3$ und $R_4$ Wasserstoff oder Methyl bedeuten.

4. Polyamide nach Anspruch 1, worin $R_1$ Methyl oder Äthyl, $R_2$ Wasserstoff, Methyl oder Äthyl, $R_3$ $C_{1-5}$-Alkyl oder unsubstituiertes Phenyl und $R_4$ Wasserstoff oder Methyl bedeuten und Z den Rest der Terephthalsäure, Isophthalsäure und/oder einer gesättigten aliphatischen Dicarbonsäure mit 6–10 C-Atomen darstellt.

5. Polyamide nach Anspruch 1, worin $R_1$ und $R_2$ Methyl, $R_3$ Isopropyl, $R_4$ Wasserstoff und Z den Rest der Terephthalsäure, Isophthalsäure oder Adipinsäure bedeuten.

6. Polyamide nach Anspruch 1, worin $R_1$ und $R_2$ Methyl, $R_3$ Isopropyl, $R_4$ Wasserstoff und Z in 50 bis 90%, besonders 60 bis 85%, der Strukturelemente der Formel I den Rest der Terephthalsäure und in 50 bis 10% der Strukturelemente der Formel I den Rest der Adipinsäure darstellen.

7. Vernetzbares Stoffgemisch, enthaltend

a) ein Polyamid nach Anspruch 1,
b) 5 bis 80 Gew.%, bezogen auf das Polyamid, eines Polythiols mit mindestens zwei Thiolgruppen pro Molekül und
c) 0,1 bis 10 Gew.%, bezogen auf das Polyamid, eines unter Lichteinwirkung radikalisch spaltbaren Initiators oder eines Photosensibilisators.

8. Stoffgemisch nach Anspruch 7, worin das Polythiol eine Verbindung der Formel VI

$$Q_1\text{-}(OCO\text{-}Q_2\text{-}SH)_{n1} \qquad (VI),$$

worin $Q_1$ -$CH_2CH_2$-, -$CH_{21}CHCH_2$- oder $C(CH_2\text{-})_4$, $n_1$ die Zahl 2, 3 oder 4 und $Q_2$ -$CH_2$-, -$CH_2CH_2$- oder -$CH_2(CH_3)$- darstellen, und insbesondere Pentaerythritol-tetrakis-(3-mercaptopropionat) ist.

9. Stoffgemisch nach Anspruch 7, worin der Photosensibilisator Benzophenon oder Thioxanthon ist.

10. Verfahren zur Herstellung transparenter Polyamide, dadurch gekennzeichnet, dass man ein Diamin der Formel II

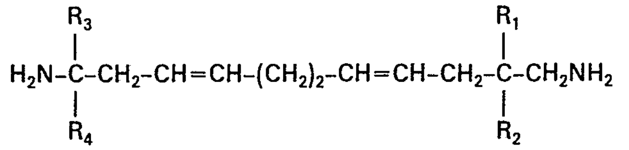

(II)

mit einer Dicarbonsäure der Formel III

HOOC-Z-COOH (III),

einem amidbildenden Derivat davon oder einem Gemisch verschiedener Dicarbonsäuren der Formel III bzw. amidbildender Derivate davon in Gegenwart einer 30 bis 1000 ppm Phosphor, bezogen auf die Summe der Kondensationskomponenten, enthaltenden Phosphorverbindung polykondensiert, wobei

$R_1$ $C_{1-12}$-Alkyl,

$R_2$ Wasserstoff oder $C_{1-12}$-Alkyl,

$R_3$ Alkyl mit 1-12 C-Atomen, Cycloalkyl mit 4-12 Ring-C-Atomen, Aralkyl mit 7 oder 8 C-Atomen, gegebenenfalls substituiertes Aryl, oder, wenn $R_4$ Wasserstoff ist, auch -CH=CH-Alkyl oder -C(Alkyl)=CH-Alkyl mit je 1-4 C-Atomen in den Alkylgruppen und

$R_4$ Wasserstoff, $C_{1-12}$-Alkyl, Cycloalkyl mit 4-12 Ring-C-Atomen, Aralkyl mit 7 oder 8 C-Atomen oder gegebenenfalls substituiertes Aryl darstellen oder

$R_1$ und $R_2$ und/oder $R_3$ und $R_4$ zusammen Alkylen mit 3-11 C-Atomen bedeuten und

Z den Rest einer gesättigten oder ungesättigten aliphatischen oder aromatischen Dicarbonsäure darstellt.

11. Verfahren nach Anspruch 10, dadurch gekennzeichnet, dass man die Verbindungen der Formel II und III in Gegenwart einer 80 bis 500 ppm Phosphor enthaltenden Phosphorverbindung polykondensiert.

12. Verfahren nach Anspruch 10, worin man eine 80 bis 500 ppm Phosphor enthaltende Phosphorverbindung verwendet, worin $R_1$ und $R_2$ Methyl sind, $R_3$ Isopropyl ist, $R_4$ Wasserstoff bedeutet und Z in 50 bis 90 Gew.% der Verbindung der Formel III den Rest der Terephthalsäure und in 50 bis 10 Gew.% der Verbindung der Formel III den Rest der Adipinsäure darstellt, wobei sich die Gewichtsprozente auf gleiche funktionelle Gruppen der Dicarbonsäuren beziehen.

13. Verfahren nach Anspruch 10, worin die Phosphorverbindung $H_3PO_4$, $NH_4H_2PO_2$ oder 2,6-Di-tert.-butyl-p-kresylphosphonsäurediäthylester ist.

**Claims**

1. A transparent polyamide which consists of recurring structural units of the formula I

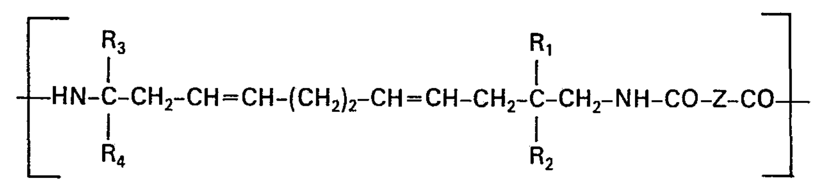

(I)

wherein $R_1$ is $C_{1-12}$alkyl, $R_2$ is hydrogen or $C_{1-12}$alkyl, $R_3$ is $C_{1-12}$alkyl, cycloalkyl having 4-12 ring C-atoms, aralkyl having 7 or 8 C-atoms, substituted or unsubstituted aryl or, if $R_4$ is hydrogen, $R_3$ is also -CH=CH-alkyl or -C(alkyl)=CH-alkyl, each having 1-4 C-atoms in the alkyl moieties, $R_4$ is hydrogen, $C_{1-12}$alkyl, cycloalkyl having 4-12 ring C-atoms, aralkyl having 7 or 8 C-atoms or substituted or unsubstituted aryl, or $R_1$ and $R_2$ and/or $R_3$ and $R_4$ together are alkylene having 3-11 C-atoms, and the symbols Z in the various structural units of the formula I are identical or different radicals of a saturated or unsaturated aliphatic or aromatic dicarboxylic acid.

2. A polyamide according to claim 1, wherein $R_1$ is $C_{1-5}$alkyl, $R_2$ is hydrogen or $C_{1-5}$alkyl, or $R_1$ and $R_2$ together are alkylene having 4-7 C-atoms, $R_3$ is $C_{1-7}$alkyl, $C_{5-8}$cycloalkyl, unsubstituted phenyl or, if $R_4$ = H, $R_3$ is also $-C(C_2H_5)=CH-CH_3$, $R_4$ is hydrogen or $C_{1-5}$alkyl and Z is the radical of terephthalic acid, of isophthalic acid, of a saturated aliphatic dicarboxylic acid having 6-12 C-atoms and/or of an unsaturated aliphatic dicarboxylic acid having 6-36 C-atoms.

3. A polyamide according to claim 2, wherein $R_3$ is $C_{1-5}$alkyl, unsubstituted phenyl or, if $R_4$ = H, $R_3$ is also $-C(C_2H_5)=CH-CH_3$, and $R_4$ is hydrogen or methyl.

4. A polyamide according to claim 1, wherein $R_1$ is methyl or ethyl, $R_2$ is hydrogen, methyl or ethyl, $R_3$ is $C_{1-5}$alkyl or unsubstituted phenyl, $R_4$ is hydrogen or methyl and Z is the radical of terephthalic acid, of isophthalic acid and/or of a saturated aliphatic dicarboxylic acid having 6-10 C-atoms.

5. A polyamide according to claim 1, wherein $R_1$ and $R_2$ are methyl, $R_3$ is isopropyl, $R_4$ is hydrogen and Z is the radical of terephthalic acid, of isophthalic acid or of adipic acid.

6. A polyamide according to claim 1, wherein $R_1$ and $R_2$ are methyl, $R_3$ is isopropyl, $R_4$ is hydrogen and Z is the radical of terephthalic acid in 50 to 90%, particularly 60 to 85%, of the structural units of the formula I and the radical of adipic acid in 50 to 10% of the structural units of the formula I.

7. A crosslinkable composition containing

a) a polyamide according to claim 1,
b) 5 to 80% by weight, based on the polyamide,

of a polythiol having at least two thiol groups per molecule, and

c) 0.1 to 10% by weight, based on the polyamide, of an initiator which can be cleaved under the action of light to give free radicals, or of a photosensitiser.

8. A composition according to claim 7, wherein the polythiol is a compound of the formula VI

$$Q_1\text{-}(OCO\text{-}Q_2\text{-}SH)_{n_1} \qquad (VI),$$

wherein $Q_1$ is $-CH_2CH_2-$, $-CH_2CHCH_2-$ or $C(CH_2-)_4$, $n_1$ is 2, 3 or 4 and $Q_2$ is $-CH_2-$, $-CH_2CH_2-$ or $-CH(CH_3)-$, and especially pentaerythritol tetrakis (3-mercaptopropionate).

9. A composition according to claim 7, wherein the photosensitiser is benzophenone or thioxanthone.

10. A process for the preparation of a transparent polyamide, which process comprises subjecting a diamine of the formula II

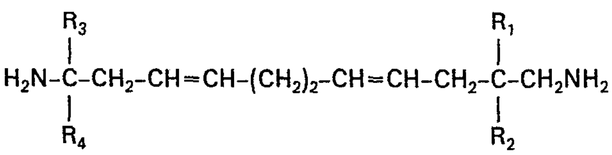

(II)

to polycondensation with a dicarboxylic acid of the formula III

$$HOOC\text{-}Z\text{-}COOH \qquad (III),$$

an amide-forming derivative thereof or a mixture of various dicarboxylic acids of the formula III or of amide-forming derivatives thereof, in the presence of a phosphorus compound containing 30 to 1,000 ppm of phosphorus, based on the sum of the condensation components, in which formulae $R_1$ is $C_{1-12}$alkyl, $R_2$ is hydrogen or $C_{1-12}$alkyl, $R_3$ is alkyl having 1-12 C-atoms, cycloalkyl having 4-12 ring C-atoms, aralkyl having 7 or 8 C-atoms, substituted or unsubstituted aryl or, if $R_4$ is hydrogen, $R_3$ is also -CH=CH-alkyl or -C(alkyl)=CH-alkyl each having 1-4 C-atoms in the alkyl moieties, $R_4$ is hydrogen, $C_{1-12}$alkyl, cycloalkyl having 4-12 ring C-atoms, aralkyl having 7 or 8 C-atoms or substituted or unsubstituted aryl, or $R_1$ and $R_2$ and/or $R_3$ and $R_4$ together are alkylene having 3-11 C-atoms, and Z is the radical of a saturated or unsaturated aliphatic or aromatic dicarboxylic acid.

11. A process according to claim 10, which process comprises, subjecting the compounds of the formulae II and III to polycondensation in the presence of a phosphorus compound containing 80 to 500 ppm of phosphorus.

12. A process according to claim 10, wherein a phosphorus compound containing 80 to 500 ppm of phosphorus is employed, and wherein in the compound of the formula II $R_1$ and $R_2$ are methyl, $R_3$ is isopropyl, $R_4$ is hydrogen and Z is the radical of terephthalic acid in 50 to 90% by weight of the compound of the formula III and the radical of adipic acid in 50 to 10% by weight of the compound of the formula III, the percentages by weight being based on identical functional groups of the dicarboxylic acids.

13. A process according to claim 10, wherein the phosphorus compound is $H_3PO_4$, $NH_4H_2PO_2$ or diethyl 2,6-di-tert-butyl-p-cresylphosphonate.

**Revendications**

1. Polyamides transparents constitués d'éléments structuraux répétés répondant à la formule I:

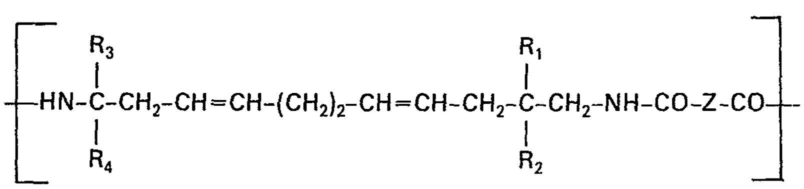

(I)

dans laquelle:

$R_1$ représente un alkyle en $C_1$-$C_{12}$,

$R_2$ représente l'hydrogène ou un alkyle en $C_1$-$C_{12}$,

$R_3$ représente un alkyle en $C_1$-$C_{12}$, un cycloalkyle contenant de 4 à 12 atomes de carbone intracycliques, un aralkyle à 7 ou 8 atomes de carbone, un aryle éventuellement substitué, ou, lorsque $R_4$ représente l'hydrogène, également un radical -CH=CH-alkyl ou -C(alkyl)=CH-alkyl dont l'alkyle ou chacun des alkyles contient de 1 à 4 atomes de carbone,

$R_4$ représente l'hydrogène, un alkyle en $C_1$-$C_{12}$, un cycloalkyle dont le cycle contient de 4 à 12 atomes de carbone, un aralkyle à 7 ou 8 atomes de carbone ou un aryle éventuellement substitué, ou encore

$R_1$ et $R_2$, et/ou $R_3$ et $R_4$, forment ensemble un alkylène contenant de 3 à 11 atomes de carbone, et

les Z représentent, dans les différents motifs de formule I, des radicaux identiques ou différents qui proviennent chacun d'un acide dicarboxylique aromatique ou aliphatique saturé ou insaturé.

2. Polyamides selon la revendication 1 dans lesquels $R_1$ représente un alkyle en $C_1$-$C_5$, $R_2$ représente l'hydrogène ou un alkyle en $C_1$-$C_5$ ou encore $R_1$ et $R_2$ forment ensemble un alkylène contenant de 4 à 7 atomes de carbone, $R_3$ représente un alkyle en $C_1$-$C_7$, un cycloalkyle en $C_5$-$C_8$ ou un phényle non substitué ou encore, dans le

cas où $R_4$ désigne H, $R_3$ peut également représenter un radical $-C(C_2H_5)=CH-CH_3$, $R_4$ représente l'hydrogène ou un alkyle en $C_1$-$C_5$, et Z représente le radical de l'acide téréphtalique, de l'acide isophtalique, d'un acide dicarboxylique aliphatique saturé contenant de 6 à 12 atomes de carbone et/ou d'un acide dicarboxylique aliphatique insaturé contenant de 6 à 36 atomes de carbone.

3. Polyamides selon la revendication 2 dans lesquels $R_3$ représente un alkyle en $C_1$-$C_5$, un phényle non substitué ou, lorsque $R_4$ désigne H, $R_3$ peut également désigner un radical $-C(C_2H_5)=CH-CH_3$, et $R_4$ représente l'hydrogène ou un méthyle.

4. Polyamides selon la revendication 1 dans lesquels $R_1$ représente un méthyle ou un éthyle, $R_2$ représente l'hydrogène, un méthyle ou un éthyle, $R_3$ représente un alkyle en $C_1$-$C_5$, ou un phényle non substitué, $R_4$ représente l'hydrogène ou un méthyle et Z représente le radical de l'acide téréphtalique, de l'acide isophtalique et/ou d'un acide dicarboxylique aliphatique saturé contenant de 6 à 10 atomes de carbone.

5. Polyamides selon la revendication 1 dans lesquels $R_1$ et $R_2$ représentent chacun un méthyle, $R_3$ représente un isopropyle, $R_4$ représente l'hydrogène et Z représente le radical de l'acide téréphtalique, de l'acide isophtalique ou de l'acide adipique.

6. Polyamides selon la revendication 1 dans lesquels $R_1$ et $R_2$ représentent chacun un méthyle, $R_3$ représente un isopropyle, $R_4$ représente l'hydrogène et Z représente, dans 50 à 90% des éléments structuraux de formule I, en particulier dans 60 à 85% de ceux-ci, le radical de l'acide téréphtalique et, dans 50 à 10% des éléments structuraux de formule I, le radical de l'acide adipique.

7. Mélanges réticulables contenant:

a) un polyamide selon la revendication 1,

b) de 5 à 80% en poids, par rapport au polyamide, d'un polythiol renfermant au moins deux radicaux mercapto par molécule et

c) de 0,1 à 10% en poids, par rapport au polyamide, d'un amorceur susceptible de subir une coupure radicalaire sous l'action de la lumière ou d'un photosensibilisateur.

8. Mélange selon la renvendication 7 dans lequel le polythiol est un composé répondant à la formule VI

$$Q_1\text{-}(OCO\text{-}Q_2\text{-}SH)_{n_1} \qquad (VI)$$

dans laquelle $Q_1$ représente un radical $-CH_2CH_2-$, $-CH_2CHCH_2-$ ou $C(CH_2-)_4$, $n_1$ représente un nombre égal à 2, à 3 ou à 4, et $Q_2$ représente un radical $-CH_2-$, $-CH_2CH_2-$ ou $-CH(CH_3)-$, et plus particulièrement le tétrakis-(mercapto-3 propionate) du pentaérythritol.

9. Mélange selon la revendication 7 dans lequel le photosensibilisateur est la benzophénone ou la thioxanthone.

10. Procédé pour préparer des polyamides transparents, procédé caractérisé en ce qu'on polycondense une diamine de formule II:

$$H_2N-\underset{R_4}{\overset{R_3}{C}}-CH_2-CH=CH-(CH_2)_2-CH=CH-CH_2-\underset{R_2}{\overset{R_1}{C}}-CH_2NH_2 \qquad (II)$$

avec un acide dicarboxylique de formule III:

$$HOOC\text{-}Z\text{-}COOH \qquad (III),$$

un dérivé amidogène d'un tel acide ou un mélange de différents acides dicarboxyliques de formule III ou de différents dérivés amidogènes de ceux-ci, en présence d'un composé du phosphore contenant de 30 à 1000 ppm de phosphore par rapport à la somme des composantes de condensation, les différents symboles présents dans les formules précédentes ayant les significations suivantes:

$R_1$ représente un alkyle en $C_1$-$C_{12}$,

$R_2$ représente l'hydrogène ou un alkyle en $C_1$-$C_{12}$,

$R_3$ représente un alkyle en $C_1$-$C_{12}$, un cycloalkyle contenant de 4 à 12 atomes de carbone dans son cycle, un aralkyle à 7 ou 8 atomes de carbone, un aryle éventuellement substitué, ou, lorsque $R_4$ désigne l'hydrogène, $R_3$ peut également représenter un radical -CH=CH-alkyl ou un radical -C(alkyl)=CH-alkyl dont l'alkyle ou chacun des alkyles contient de 1 à 4 atomes de carbone,

$R_4$ représente l'hydrogène, un alkyle en $C_1$-$C_{12}$, un cycloalkyle contenant de 4 à 12 atomes de carbone dans son cycle, un aralkyle à 7 ou 8 atomes de carbone ou un aryle éventuellement substitué, ou encore

$R_1$ et $R_2$, et/ou $R_3$ et $R_4$, forment ensemble un alkylène contenant de 3 à 11 atomes de carbone, et

Z représente le radical d'un acide dicarboxylique aliphatique, saturé ou insaturé, ou d'un acide dicarboxylique aromatique.

11. Procédé selon la revendication 10 caractérisé en ce qu'on polycondense les composés de formules II et III en présence d'un composé du phosphore contenant de 80 à 500 ppm de phosphore.

12. Procédé selon la revendication 10 caractérisé en ce qu'on utilise un composé du phosphore contenant de 80 à 500 ppm de phosphore et en ce que $R_1$ et $R_2$ représentent chacun un méthyle, $R_3$ représente un isopropyle, $R_4$ l'hydrogène et Z, dans 50 à 90% en poids du composé de formule III, le radical de l'acide téréphtalique et, dans 50 à 10% en poids du composé de formule III, le radical de l'acide adipique, les pourcentages pondéraux étant relatifs à des radicaux fonctionnels identiques des acides dicarboxyliques.

13. Procédé selon la revendication 10 caractérisé en ce que le composé du phosphore est $H_3PO_4$, $NH_4H_2PO_2$ ou le (di-tert-butyl-2,6 p-crésyl)-phosphonate de diéthyle.